Europäisches Patentamt

European Patent Office

Office européen des brevets

(11) Publication number: 0 381 813
A2

# (12) EUROPEAN PATENT APPLICATION

(21) Application number: 89116896.5

(22) Date of filing: 12.09.89

(51) Int. Cl.⁵: A61B 5/00

(30) Priority: 06.02.89 JP 27211/89

(43) Date of publication of application:
16.08.90 Bulletin 90/33

(84) Designated Contracting States:
DE GB

(71) Applicant: FUKUDA DENSHI CO., LTD.
39-4, Hongo 3-chome Bunkyo-ku
Tokyo 113(JP)

(72) Inventor: Maruyama, Mitsuya c/o Fukuda
Denshi Co., Ltd.
Hongo enterprise place 2-35-8 Hongo
Bunkyo-ku Tokyo(JP)

(74) Representative: Behn, Klaus, Dipl.-Ing.
Patentanwalt Lindenberg 34
D-8134 Pöcking bei München(DE)

(54) Multifunctional type apparatus for monitoring the patient.

(57) A multifunctional type apparatus for monitorring the the patient, comprising an one apparatus 1 which has a plurality of a first monitor M1, a second monitor M2, • • •, and a n th monitor Mn, said monitors monitorring each living body signal S of patient, and having respectively different functions, which functions are each exchangeable.

EP 0 381 813 A2

## MULTIFUNCTIONAL TYPE APPARATUS FOR MONITORRING THE PATIENT

### BACKGROUND OF THE INVENTION

#### 1. Field of the Invention

The present invention relates to a multifunctional type apparatus for monitorring the patient. More particularly, it relates to a multifunctional type apparatus for monitorring the patient in which one apparatus has many functions exchangeable according to uses thereof.

#### 2. Description of the Related Art

Generally, an apparatus for monitorring the patient is defined an apparatus which monirors living body signals composed of electrocardiograms etc. of patients housed in ICU or CCU etc..

Conventionally, there are a multiparameter central monitor, bedside monitor and ECG central monitor, as concrete apparatuses for monitorring the patient.

The multiparameter central monitor is an apparatus for monitorring living body signals composed of electrocardiogram, heart beat, and blood pressure etc. lead out from a plurality of patients, which monitor is setted in a nurse center, for example.

The bedside monitor is an apparatus for monitorring living body signals composed of electrocardiogram, heat, and blood pressure etc. of specific patients, which is setted by the side of the beds of them.

The ECG central monitor is an apparatus for monitorring only the electrocardiogram of living body signals of a plurality of patents, which is settled in a nurse center, for example.

However, the multiparameter central monitor, bedside monitor and ECG central monitor are constituted separately.

Therefore, the manufacturer of the above three monitors should prepare parts thereof or processes, separately, which is unsuitable for mass production.

And before the above monitors are completed, the stock thereof should be managed, separalely.

That is to say, the prior are has the defect that the efficiency of the production and salling is bad.

On the other hand, the functions of the above monitors a re so different that they have no combined use each other.

Accordingly, when one of the three monitors is trouble, the user thereof cannot divert one of the other monitors to the trouble monitor.

That is to say, the prior art has the defect that the efficiency of diagnois with the monitor is low.

### SUMMARY OF THE INVENTION

An object of the present invention is to improve the efficiency of manufacturing, selling, and diagnois, regaring the apparatus for monitorring the patient.

The above-mentioned object can be achieved object can be achieved by a multifunctional type apparatus for monitorring the the patient, comprising an one apparatus which has a plurality of a first monitor, a second monitor, . . , and a n th monitor, said monitors monitorring each living body signal of patient, and having respectively different functions, which functions are each exchangeable.

### BRIEF DESCRIPTION OF THE DRAWINGS

Further features and advantages of the present invention will be apparent from the ensuring dencription with reference to the accompanying drawings, wherein:

Fig. 1 is a drawing of the principle of the present invention;

Fig. 2 is a drawing of the embodiment of the present invrntion;

Fig. 3 is a drawing of one applicalication of the present invention;

Fig. 4 is a drawing of another application of the present invention.

### DESCRIPTION OF THE PREFERRED EMBODIMENS

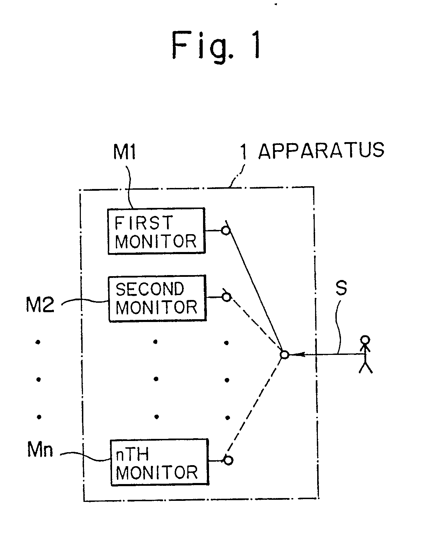

Figure 1 is a drawing of the principle of the present invention, wherein reference numeral 1 shows an one apparatus, M1 a first monitor, M 2 a second monitor • • •, Mn a n th monitor, S a living body signal.

The first monitor M1, the second monitor M2, • • •, and the n th monitor Mn are respectively arranged in the one apparatus 1.

The first monitor M1 to the n th monitor Mn monitor the living body signal S of the patient, each and have different functions, respectively, which functions are exchangeable.

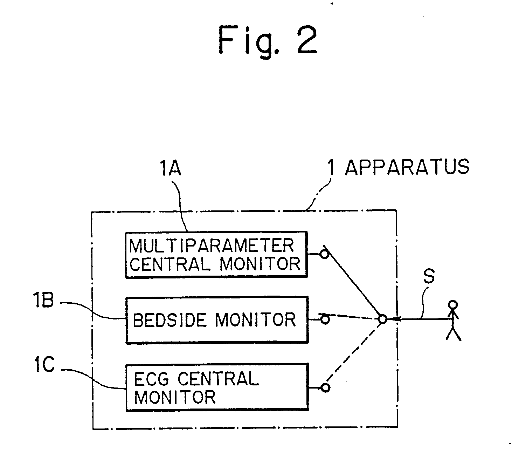

Figure 2 is a drawing of the embodiment of the present invention, wherein reference numeral 1 shows an apparatus, 1A a multiparameter central monitor, 1B a bedside mlnitor, 1C a ECG cental monitor.

The above three minitors have diffent functions, each as mentioned in in "2. Description of the Related Art of BACKGROUND OF THE INVENTION".

That is to say, the functions thereof are follows.

The multiparameter central monitor is an apparatus for monitorring living body signals composed of electrocardiogram, heart beat, and blood pressure etc. lead out from a plurality of patients, which monitor is setted in a nurse center, for example.

The bedside monitor is an apparatus for monitorring living body signals composed of electrocardiogram, heat, and blood pressure etc. of specific patients, which is setted by the side of the beds of them.

The ECG central monitor is an apparatus for monitorring only the electrocardiogram of living body signals of a plurality of patents, which is settedin a nurse center, for example.

The above three monitors 1A to 1C are assembled into the one apparatus 1, functions of which monitors are exchangeabe, for example, by using soft ware.

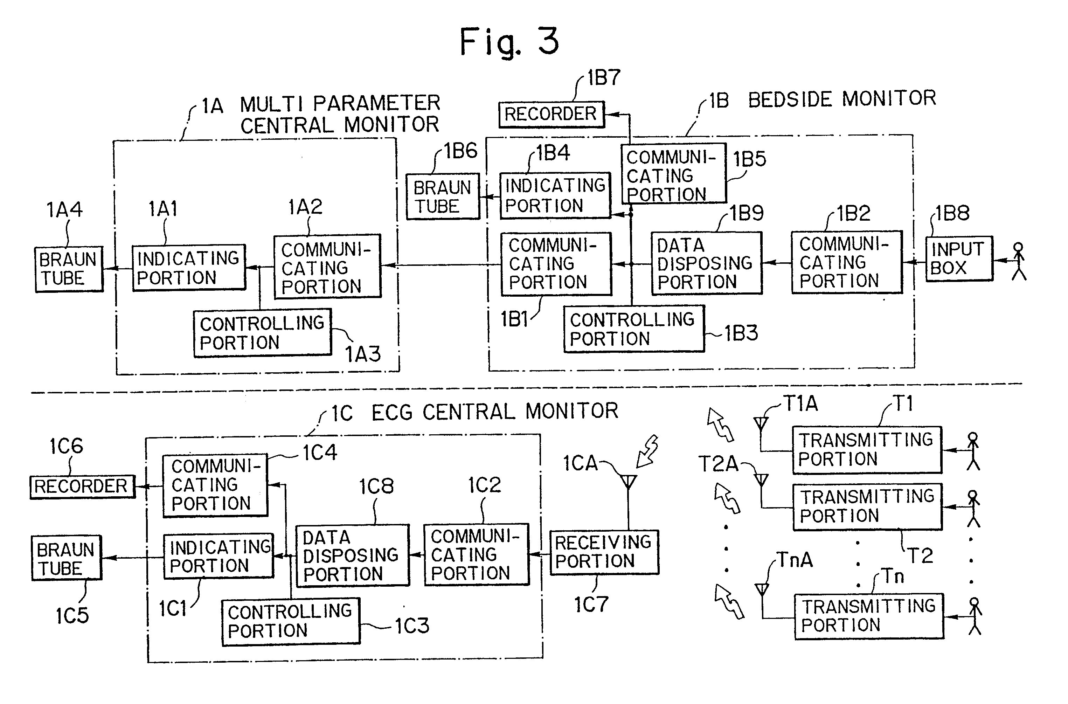

Figure 3 is a drawing of one application of the present invention.

The operation of the present invention will be described hereinafter with reference to Fig 3.

First, the upper part of Fig.3 with respect to the dotted line will be described, wherein two multifunctional type apparatuses for monitorrong the patient are applied.

One of two multifunctional apparatuses for monitorring the patient is switched to the multiparameter central monitor 1A of three monitors 1A to 1C (see Fig, 2 ), and the other is switched to the bedside monitor 1A of the above three monitors.

The above switching operation is carried out with a switching element (omited in the drawings) mounted on the rear of each apparatus, in the surrounding of which switching element there are arranged numerals 0, 1, and 2, etc..

For example, when the switching element is setted by the numeral 0, the apparatus 1 (see Fig, 2) is switched to the monitor 1A, and when it is setted by the numeral 2, the apparatus 1 is switched to the bedside monitor 1 B.

The multiparameter central monitor 1A is setted in the nurse center, and the bedside monitor 1B is setted by the side of the bed of the patient, which monitors 1A and 1B are connected with each other by a cable.

The multiparameter central monitor 1A is composed of an indicating portion 1A1, a communicating portion 1A2 and a controlling portion 1A3.

The bedside monitor 1B is provided with an indicating portion 1B4, communicating portions 1B1, 1B5 and 1B2, a controlling portion 1B3, and a data disposing portion 1B9, which monitor may be switched freely to a monitor with two beds or a monitor with four beds etc..

The indicating portions 1A1 and 1B4 are apparatuses for converting input data into video signals so as to indicate those data on braun tubes 1A4 and 1B6, respectively.

The communicating portion 1A2, 1B5 and 1B2 are apparatuses for transferring data, respectively.

The date disposing portion 1B9 is an apparatus for treating data according to the fixed order.

The controlling portions 1A3 and 1B3 are apparatuses for controlling the apparatuses, for example, adjusting the timing necessary to transmit data.

The living body signals of electrocardiogram etc, lead ouf from the patient, that is to say, data are input to an input box 1B8.

Data in analog form are converted into digital form by the input box 1B8, thereafter they are input to the bedside monitor 1B, wherein they are indicatid on the broun tube 1B6 through the communicating portion 1B2, the data disposing portion 1B9, and the indicating portion 1B4, as well as they are recorded on the recorder 1B7 through the communicating portion 1B2, the data disposing portion 1B9, and the communicating portion 1B5.

Data, output from the bedside monitor 1B, are input to the multiparameter central monitor 1A setted in the nurse center, and thereby they are indicated on the braun tube 1A4 through the communicating portion 1A2 and the indicating portion 1A1.

Next, the lower part of Fig.3 with respect to the dotted line will be described, wherein a radio communication system is introduced.

The switching element is adjusted to the numeral 1, so as to use the one apparatus 1 (see Fig. 2) as the ECG central monitor 1C.

In the lower part of Fig.3, transmitting portions T1, T2, • • •, Tn are electrocardiogram telemeters mounted on many patients, for example.

The ECG central monitor 1C is provided with an indicating portion 1C1, communicating portions 1C4 and 1C2, a data disposing portion 1C8, and a controlling portion 1C3.

Electrocardiogram signals are lead out from the living bodies of the patients, which signals are input to the transmitting portions T1, T2, • • • • Tn, and threby are modulated.

After the electrocardiogram signals are modulated, they are output from antennas T1A,T2A, • • • T2A to the space, and are input to an antenna 1CA of the ECG central monitor 1C.

The modulated electrocardiogram signals input to the antenna ICA, are demodulated in a receiving portion 1C7, and thereafter are input to the communicating portion 1C2.

The demodulated electrocardiogram signals, that is to say, data are output from the communicating portion 1A2 to the data disposing portion 1C8, wherein data are treated.

The data output from the data disposing portion 1C8, are indicated on a braun tube 1C5 and are recorded in a recorder 1C6 through the indicating portion 1C1 and the communicating portion 1C4, respectively.

In the lower part of Fig.3, the bedside monitor 1B is apart from.

However, the bedside monitor 1B could be applied to, by connecting the output side of the communicating portion 1B1 with the input side of the ECG central monitor 1C.

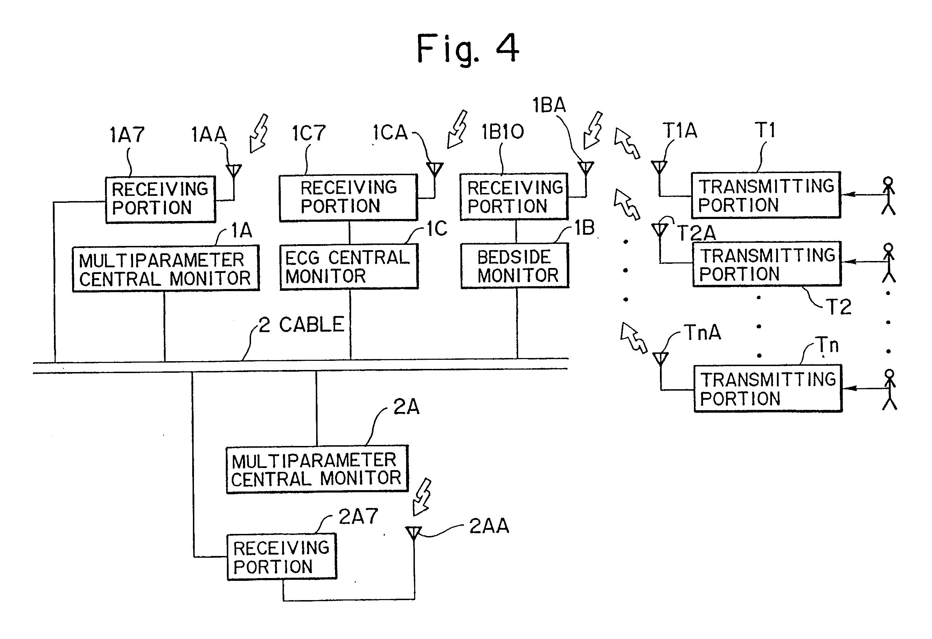

Figure 4 is a drawing of another application of the present invention, wherein multiparameter central monitors 1A and 2A, an ECG central monitor 1C1 and a bedside monitor 1B are connected with each other through a cable 2.

Another application of Figure 4 is characterized in that the multiparameter central monitor 1A and 2A are able to indicate freely, for example four beds by four beds or eight beds by eight beds, living body signals transmitted from transmitting portions T1, T2, • • •,Tn which constitute bedside monitors of many patients.

According to the present invention, in the one apparatus 1, a plurality of a first monitor M1, a second monitor M2, • • •, and an th monitor Mn are provided with, which monitors have respectively different functions exchangeable.

Hence, the above many functions in the one apparatus 1 are exchangeable according to the use thereof.

For example, the multiparameter central monitor, bedside monitor and ECG central monitor are constituted in the one apparatus.

Therefore, the maker of the one apparatus 1 could simplify parts thereof or processes, which is suitable for mass production.

And after the one apparatus is completed, the stock management thereof is able to be carried out easily, because the stock goods become to decrease in number.

That is to say, the present invention has the effect that the efficiency of the production and salling is high.

On the other hand, if the user thereof has only the one apparatuses necessary in number at a minimum, he can change them each other by means of exchanging the above functions, in case of trouble.

That is to say, the present invention has the effect that the efficiency of diagnois is high.

## Claims

1. A multifunctional type apparatus for monitorring the the patient comprising:
an one apparatus (1) which has a plurality of a first monitor (M1), a second monitor (M2), • • •, and an th monitor (Mn),
said monitors monitorring each living body signal (S) of patient, and having respectively different functions, which functions are each exchangeable.

2. A multifunctional type apparatus for monitorring the the patient according to claim 1, wherein said monitors are a multiparameter central monitor 1A for monitorring living body signals of a plurality of patients, a bedside monitor 1B for monitorring living body signals of specific patients, and an ECG central monitor 1C for monitorring only the electrocardiograms of living body signals of a plurality of patients.

# Fig. 1

# Fig. 2

# Fig. 3

1A MULTI PARAMETER CENTRAL MONITOR

1B BEDSIDE MONITOR

1B7 RECORDER

1B6 BRAUN TUBE

1B4 INDICATING PORTION

1B5 COMMUNI-CATING PORTION

1A4 BRAUN TUBE

1A1 INDICATING PORTION

1A2 COMMUNI-CATING PORTION

1A3 CONTROLLING PORTION

1B1 COMMUNI-CATING PORTION

1B9 DATA DISPOSING PORTION

1B2 COMMUNI-CATING PORTION

1B8 INPUT BOX

1B3 CONTROLLING PORTION

1C ECG CENTRAL MONITOR

1C6 RECORDER

1C4 COMMUNI-CATING PORTION

1C8 DATA DISPOSING PORTION

1C2 COMMUNI-CATING PORTION

1CA

1C5 BRAUN TUBE

1C1 INDICATING PORTION

1C3 CONTROLLING PORTION

1C7 RECEIVING PORTION

T1A T1 TRANSMITTING PORTION

T2A T2 TRANSMITTING PORTION

TnA Tn TRANSMITTING PORTION

EP 0 381 813 A2

# Fig. 4

RECEIVING PORTION — 1A7, 1AA

RECEIVING PORTION — 1C7, 1CA

RECEIVING PORTION — 1B1O, 1BA

TRANSMITTING PORTION — T1A, T1

MULTIPARAMETER CENTRAL MONITOR — 1A

ECG CENTRAL MONITOR — 1C

BEDSIDE MONITOR — 1B

TRANSMITTING PORTION — T2A, T2

2 CABLE

TRANSMITTING PORTION — TnA, Tn

MULTIPARAMETER CENTRAL MONITOR — 2A

RECEIVING PORTION — 2A7, 2AA

EP 0 381 813 A2